(19) Europäisches Patentamt
European Patent Office
Office européen des brevets

(11) Numéro de publication: **0 202 157**
**B1**

# (12) FASCICULE DE BREVET EUROPÉEN

(45) Date de publication du fascicule du brevet: **23.08.89**

(51) Int. Cl.⁴: **C 07 D 277/42**, C 07 D 277/56, C 07 D 417/12, A 61 K 31/425, A 61 K 31/44

(21) Numéro de dépôt: **86400959.2**

(22) Date de dépôt: **30.04.86**

(54) **Amino-2 thiazoles N-substitués, leur procédé de préparation et leur application en thérapeutique.**

(30) Priorité: **30.04.85 FR 8506568**

(43) Date de publication de la demande: **20.11.86 Bulletin 86/47**

(45) Mention de la délivrance du brevet: **23.08.89 Bulletin 89/34**

(84) Etats contractants désignés: **AT BE CH DE FR GB IT LI LU NL SE**

(56) Documents cité:
**DE-A-2 309 251**
**FR-A-1 302 433**
**GB-A-2 022 085**

(73) Titulaire: **LABORATOIRES CHAUVIN S.A., 104 Rue de la Galéra, F-34000 Montpellier (FR)**

(72) Inventeur: **Coquelet, Claude, 113 Rue des Lilas, F-34980 St Gely du Fesc (FR)**
Inventeur: **Sincholle, Daniel 343 Avenue de la Trémoulette, St Clement la Riviere, F-34270 St Mathieu de Treviers (FR)**
Inventeur: **Bonne, Claude, Allée des Ifs, Castelnau le Lez (FR)**
Inventeur: **Alazet, Alain, 5 Impasse des Vignerons, F-34000 Agde (FR)**

(74) Mandataire: **Bressand, Georges, c/o CABINET LAVOIX 2 Place d'Estienne d'Orves, F-75441 Paris Cedex 09 (FR)**

**EP 0 202 157 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne de nouveaux amino-2 thiazoles N-substitués ayant une activité antiinflammatoire et antiallergique. Ces composés peuvent être utilisés notamment dans le traitement des phénomènes inflammatoires en particulier dans le traitement des phénomènes inflammatoires oculaires.

La réponse inflammatoire oculaire est la séquence des événements entraînés par une lésion ou une irritation des tissus de l'oeil. Elle se traduit par l'augmentation de la pression intraoculaire, une infiltration leucocytaire dans les tissus et les liquides de l'oeil, une augmentation du taux de protéines dans l'humeur aqueuse et une uvéite.

Bien que la nature de la réponse inflammatoire soit extrêmement variée et ses médiateurs multiples (kinines, histamine, etc..) il y a toujours production de métabolites de l'acide arachidonique proinflammatoires au niveau du tissu lésé, à savoir prostaglandines (PGs) et hydroxy-acides eicosatétraénoïques (HETEs). Cette production est augmentée par l'infiltration des leucocytes polymorphonucléaires (PMNs) vers le lieu de l'inflammation, qui produisent à leur tour durant la phagocytose des PGs et des HETEs. Les effets biologiques connus des HETEs sont, entre autres, le chimiocinétisme et le chimiotactisme vis-à-vis des PMNs.

Les PGs et les HETEs sont des métabolites de l'acide arachidonique acide gras en $C_{20}$. Ils sont synthétisés selon deux voies distinctes:

- la voie de la cyclooxygénase conduit successivement aux endoperoxydes $PGG_2$ et $PGH_2$ précurseurs des divers PGs de la série 2. Parmi celles-ci, $PGE_2$ et $PGI_2$ (prostacycline) sont particulièrement vasodilatatrices;
- la voie de la lipoxygénase conduit aux acides non cyclisés mono- et dihydroxyeicosatétraénoïques (HETEs) qui sont pour la plupart des agents proinflammatoires, comme leurs précurseurs, les acides hydroxyperoxylés correspondants (HPETEs). Ainsi, le 5,12-diHETE ou leucotriène $B_4$ ($LTB_4$) est l'agent chimiotactique le plus puissant connu pour les leucocytes humains. Des métabolites de cette voie sont impliqués en outre dans les réactions allergiques telles que l'asthme.

Contrairement aux PGs qui ont des précurseurs communs, $PGG_2$ et $PGH_2$, les produits de lipoxygénase ont des précurseurs différents:

- le 12-HPETE dans les plaquettes, synthétisé sous l'action d'une 12-lipoxygénase, conduit à la formation de 12-HETE;
- dans les leucocytes, 5-HPETE et 15-HPETE sont principalement invoqués, conduisant aux 5-HETE, 5,12-diHETE et 15-HETE.

La recherche d'agents thérapeutiques susceptibles de s'opposer à la réaction inflammatoire était orientée vers les voies suivantes:

- inhibition de la libération d'acide arachidonique par les glucocorticoïdes. L'inconvénient majeur des glucocorticoïdes est de provoquer une augmentation de la pression intraoculaire et de favoriser l'apparition d'un glaucome ou d'une cataracte;
- inhibition de la voie de la cyclooxygénase par des antiinflammatoires non stéroïdiens tels que Aspirine, Indométacine, Tandéril. Mais des études expérimentales récentes montrent que l'Indométacine augmente l'infiltration leucocytaire dans l'humeur aqueuse: en inhibant la voie de la cyclooxygénase, elle favorise la voie de la lipoxygénase et donc la libération de produits chimiotactiques.

L'inhibition simultanée de la voie de la 5-lipoxygénase et de la cyclooxygénase est susceptible de bloquer la synthèse des différents médiateurs proinflammatoires comme le font les glucocorticoïdes mais sans en présenter les effets gênants.

De plus l'inhibition de la voie 5-lipoxygénase est susceptible de bloquer la synthèse des leucotriènes peptidiques LTC4 et son métabolite LTD4 (anciennement connus sous le nom SRS-A) qui jouent un rôle pathophysiologique important dans les réactions d'hypersensibilité immédiate.

La présente invention est précisément basée sur la découverte que certains amino 2-thiazoles inhibent à la fois la lipoxygénase leucocytaire et la formation des prostanoïdes plaquettaires. Ils s'opposent donc au développement de la phase vasculaire de l'inflammation (impliquant les HETEs).

La présente invention a ainsi pour objet des composés de formule:

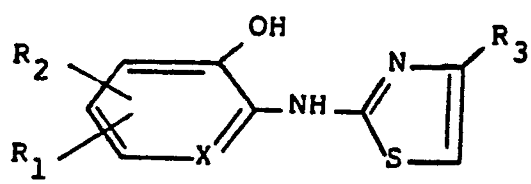

(I)

dans laquelle:

X représente un groupe C - H ou un atome d'azote,

$R_1$ et $R_2$ représentent indépendamment l'un de l'autre un atome d'hydrogène, un atome d'halogène, un radical alkyle en $C_1$-$C_{12}$ un radical alkoxy en $C_1$-$C_6$, un radical alkylthio en $C_1$-$C_6$, un radical alkenyloxy en $C_2$-$C_6$ un radical cycloalkyle en $C_5$-$C_7$, un radical trifluorométhyle, un groupe nitro un groupe amino un groupe (alkyl en $C_1$-$C_6$) amino ou un groupe di(alkyl en $C_1$-$C_6$) amino,

$R_3$ représente un atome d'hydrogène un groupe $-CH_2OH$ ou un groupe $-COOR_4$ $R_4$ étant un atome d'hydrogène ou un groupe alkyle en $C_1$-$C_6$,

ainsi que leurs sels pharmaceutiquement acceptables.

La présente invention a également pour objet des compositions thérapeutiques contenant à titre de principe actif un composé de formule I ou l'un de ses sels pharmaceutiquement acceptables.

Par sels pharmaceutiquement acceptables on désigne des sels d'addition que forment les composés de formule (I) avec des acides pharmaceutiquement acceptables, ainsi que les sels que forment les composés de formule (I) à groupe acide ($R_3$ = COOH) avec des bases pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide lactique l'acide succinique, l'acide tartrique et l'acide pamoïque.

De même, "les sels avec des bases pharmaceutiquement acceptables" désignent les sels qui ne modifient pas les propriétés biologiques des acides libres. Ces sels peuvent être notamment ceux formés avec des bases minérales, telles que l'hydroxyde de sodium, l'hydroxyde de potassium, l'hydroxyde de lithium, l'hydroxyde de calcium, l'hydroxyde de magnésium, ou des bases organiques telles que la glucamine, la N-méthylglucamine, la N,N-diméthylglucamine, l'éthanolamine, la diéthanolamine, la morpholine, la N-méthyl morpholine et la tris -(hydroxyméthyl)-méthylamine.

Le terme "halogène" désigne le fluor, le chlore, le brome ou l'iode.

Les groupes alkyle ont de préférence de 1 à 6 atomes de carbone.

Une classe préférée de composés de formule I est celle dans laquelle $R_1$ est un atome d'hydrogène, un atome d'halogène en position 5, un groupe alkyle en $C_1$-$C_4$ en position 4 ou 5 et $R_2$ est un atome d'hydrogène ou bien dans laquelle $R_1$ et $R_2$ sont des groupes alkyle en $C_1$-$C_4$ en positions 3 et 5.

Les composés de formule I dans laquelle $R_3$ est un atome d'hydrogène ou un groupe $-COOR_4$, $R_4$ étant un groupe alkyle en $C_1$-$C_6$, peuvent être préparés par réaction d'une thiourée N-substituée de formule:

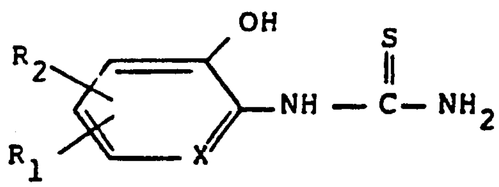

(II)

dans laquelle $R_1$, $R_2$ et X ont la définition donnée ci-dessus, avec du dichloro-1,2 éthoxy-2 éthane, du chloroacétaldéhyde, du chloroacétaldéhyde diéthylacétal de l'acétate de dichloro-1,2 éthyle ou un bromopyruvate d'alkyle en $C_1$-$C_6$.

La réaction d'une thiourée N-substituée de formule II avec par exemple le chloroacétaldéhyde diéthylacétal peut être effectuée en portant au reflux pendant 30 minutes à 24 heures une solution alcoolique de quantités équimolaires de réactifs en présence d'un catalyseur acide tel que l'acide chlorhydrique ou l'acide p-toluène sulfonique.

Les thiourées de formule II peuvent être préparées par action du thiocyanate de benzoyle sur une amine de formule:

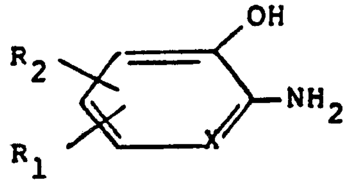

(III)

dans laquelle $R_1$, $R_2$ et X ont la définition donnée ci-dessus.

Cette réaction peut être effectuée par exemple en ajoutant lentement une solution acétonique d'amine de formule III à une solution acétonique portée au reflux de thiocyanate de benzoyle préparé in-situ selon un procédé connu. Après un bref temps de reflux le produit de condensation est hydrolysé par une solution aqueuse alcaline pour conduire à un dérivé de formule II.

Les composés de formule I dans laquelle $R_3$ est un groupe -COOH peuvent être obtenus par saponification des esters d'alkyle correspondants.

Les composés de formule I dans laquelle $R_3$ est un groupe, $-CH_2OH$ peuvent être obtenus par réduction de

ces esters, par exemple par l'hydrure de lithium aluminium.

Les sels d'addition des composés de formule I avec des acides pharmaceutiquement acceptables peuvent être préparés de manière classique par réaction des bases libres avec un acide ou sel d'acide notamment en solution éthérée ou alcoolique. Les sels des composés à groupe acide avec des bases s'obtiennent par réaction de l'acide avec une base dans un solvant usuel.

On donnera ci-après dans le tableau I des exemples de composés de formule I. Les exemples suivants illustrent la préparation des composés de formule I.

**Exemple 1:**

a) Préparation de l'(hydroxy-2 méthyl-4 phényl)- 1 thio-2 urée

On ajoute goutte à goutte 0,2 mole de chlorure de benzoyle à une solution de 0,22 mole de thiocyanate d'ammonium dans 100 ml d'acétone anhydre. On porte à reflux 5 minutes après la fin de l'addition puis on ajoute goutte à goutte, de façon à maintenir un léger reflux, une solution de 0,2 mole d'amino-6 m-crésol dans 50 ml d'acétone anhydre. On poursuit l'agitation et le chauffage pendant 5 minutes après l'addition, on verse le mélange réactionnel dans 1,5 l d'eau glacée, on essore et on met en suspension les cristaux obtenus dans 300 ml d'une solution de soude à 10 %. On porte à ébullition pendant 10 minutes, on filtre la solution sur laine de verre et l'acidifie fortement avec de l'acide chlorhydrique concentré. On ramène la solution à pH 8 avec de l'ammoniaque. Les cristaux qui précipitent par refroidissement sont essorés, lavés avec de l'eau et recristallisés dans l'éthanol (F: 175 - 7° C) Rdt: 65 %.

b) Préparation du chlorhydrate d'(hydroxy-2 méthyl-4 phényl) amino-2 thiazole.

On porte à reflux pendant 12 heures un mélange de la thiourée obtenue en a (0,1 mole) de chloroacétaldéhyde diéthylacétal (0,1 mole), d'acide p.toluène sulfonique (0,015 mole) dans 300 ml d'alcool à 70°. Après évaporation sous vide du solvant, on triture le résidu huileux dans une solution aqueuse d'acétate de sodium. On extrait la phase organique avec de l'éther, on lave la solution éthérée avec de l'eau, on la sèche sur sulfate de sodium anhydre puis on la décolore avec du charbon. Après filtration la solution éthérée est saturée par un courant d'acide chlorhydrique gazeux sec. Les cristaux formés sont isolés puis recristallisés dans un mélange éthanol-éther (F: 184 - 6° C) Rdt: 50 %.

**Exemple 2:**

Préparation du bromhydrate d'(hydroxy-2 méthyl-4 phényl) amino-2 carbéthoxy-4 thiazole.

On porte à reflux pendant 3 heures 0,065 mole d'(hydroxy-2 méthyl-4 phényl)-1 thio-2 urée et 0,077 mole de bromopyruvate d'éthyle dans 100 ml d'éthanol à 95°. Après évaporation du solvant, on fait cristalliser l'huile résiduelle dans l'éther diéthylique. On recristallise ensuite dans un mélange éthanol-éther. (F: 158 - 60° C). Rendement: 65 %.

**Exemple 3:**

(hydroxy-2 méthyl-4 phényl) amino-2 hydroxy-méthyl-4 thiazole

On ajoute, par petites fractions à température ambiante, 0,02 mole du produit obtenu à l'exemple 2 à une suspension de 0,05 mole d'hydrure de lithium aluminium dans 100 ml de THF anhydre. On maintient l'agitation pendant 1 heure à température ambiante, refroidit et détruit l'excès d'hydrure par addition d'eau, on essore et évapore sous vide le filtrat au bain-marie. Le résidu huileux est ensuite cristallisé dans de l'éther et recristallisé dans un mélange méthanol-éther. (F: 188 - 90° C). Rendement: 30 %.

**Exemple 4:**

Chlorhydrate de l'acide (hydroxy-2 méthyl-4 phényl) amino-2 thiazole carboxylique-4

On porte à reflux pendant 2 heures 0,03 mole de bromhydrate d'(hydroxy-2 méthyle-4 phényl) amino-2 carbéthoxy-4 thiazole dans 100 ml d'une solution de soude 1 N. Après refroidissement, on précipite le chlorhydrate de l'acide par addition d'acide chlorhydrique concentré. On recristallise dans l'éthanol. (F: 248 - 50° C). Rendement: 60 %.

**Tableau I**
Composés de formule I

| Composé n° | $R_1$ | $R_2$ | X | $R_3$ | Rendement (%) | Fusion (°C) | Solvant de recristallisation | Sel |
|---|---|---|---|---|---|---|---|---|
| 1 | H | H | CH | H | 40 | 159 - 61 | Acétone-éthanol | Chlorhydrate |
| 2 | 5-Cl | H | CH | H | 30 | 172 - 4 | Acétone | Chlorhydrate |
| 3 | 4-$CH_3$ | H | CH | H | 50 | 184 - 6 | Ethanol | Chlorhydrate |
| 4 | 5-$CH_3$ | H | CH | H | 40 | 162 - 4 | Ethanol | Chlorhydrate |
| 5 | FIG | H | CH | H | 25 | 172 - 4 | Acétone-méthanol | Chlorhydrate |
| 6 | 3-CH | 5-$CH_3$ | CH | H | 40 | 209 - 11 | Ethanol | Chlorhydrate |
| 7 | H | H | N | H | 20 | 238 - 40 | Méthanol-éther | Chlorhydrate |
| 8 | 4-$CH_3$ | H | CH | -$COOC_2H_5$ | 65 | 158 - 60 | Ethanol-éther | Bromhydrate |
| 9 | 4-$CH_3$ | H | CH | -$CH_2OH$ | 30 | 188 - 90 | Ether | |
| 10 | 4-$CH_3$ | H | CH | -COOH | 60 | 248 - 50 | Ethanol | Cholohydrate |
| 11 | 5-$CH_3$ | H | CH | -COOH | 70 | 253 - 55 | Ethanol | Chlorhydrate |
| 12 | 5-$OCH_3$ | H | CH | H | 54 | 180 - 82 | Méthanol | |

On donnera ci-après des résultats des études pharmacologiques et toxicologiques mettant en évidence les propriétés des composés de formule I.

**1 - Pharmacologie biochimique "in vitro"**

**1.1** - Inhibition de la lipoxygénase des leucocytes circulants humains (PMNs) par radioimmunodosage du LTC4

Méthodes

- Préparation d'une suspension leucocytaire

On fait sédimenter pendant 1 heure à 37°C du sang humain hépariné dans une solution saline stérile contenant 4,5 % de Dextran T 500. La phase supérieure riche en leucocytes est collectée, déposée sur du Ficoll Paque (7/3 v/v) puis centrifugée (20 minutes 400 x g, 4°C). Le culot de centrifugation est traité avec du NH4 Cl 0,75 % pour lyser les hématies. Après centrifugation (20 minutes, 400 x g, 4°C) on élimine le surnageant et remet le culot en suspension dans un tampon PBS Dulbecco contenant 1 mM de chlorure de calcium. On ajuste le volume de la suspension pour avoir la concentration leucocytaire souhaitée.

- Radioimmunodosage du LTC4

On préincube pendant 2 minutes la suspension leucocytaire avec le composé de formule I à tester, incube ensuite pendant 8 minutes à 37°C en présence d'ionophore calcique A23187 (0,25 µM). On bloque l'incubation par addition d'un volume d'eau glacée et centrifuge (15 minutes 1500 x g, 4°C). Des aliquots du surnagéant sont ensuite soumis au radioimmunodosage (KIT New England Nuclear).

Résultats

Le pourcentage d'inhibition de la voie 5-lipoxygénase est mesuré en fonction de la concentration de chaque composé. De ces courbes on déduit la concentration inhibant 50 % de formation du traceur LTC4 (CI50).

A titre d'exemple, un certain nombre de résultats sont donnés dans le tableau II.

1.2 - Inhibition du métabolisme du $14_C$-Arachidonate dans les plaquettes de lapin

Méthodes

- Préparation d'un plasma riche en plaquettes (PRP)

On prélève en intracardiaque 12 ml de sang sur ACD (anticoagulant constitué par une solution aqueuse contenant 0,27 g d'acide citrique, 0,5 g de citrate disodique et 0,02 g de glucose pour 20 ml d'eau), à raison de 1 volume d'ACD pour 6 volumes de sang. On centrifuge (10 minutes, 200 x g, 20°C), collecte le surnageant (PRP) et le recentrifuge (15 minutes, 1500 x g, 20°C). On lave le culot plaquettaire dans le tampon I (NaCl: 8 g/l, Tris: 1,21 g/l, KCl: 0,2 g/l, Gélatine: 2,5 g/l; on chauffe à 90°C pour dissoudre la gélatine, puis ajoute glucose: 1 g/l, acide N,N'-éthylèneglycol bis (β-aminoéthyl) éther tétraacétique ou EGTA: 0,075 g/l). On centrifuge la suspension (15 minutes 1500 x g, 20°C) et reprend le culot dans le tampon II (NaCl: 8 g/l, Tris: 1,21 g/l, KCl: 0,2 g/l, $CaCl_2$: 0,145 g/l, $MgCl_2$: 0,2 g/l, gélatine: 2,5 g/l; on porte à 90°C, ajoute glucose: 1 g/l, ajuste à pH 7,4 avec HCl 0,1 N).

- Métabolisme du $14_C$-Arachidonate

On préincube la suspension plaquettaire (0,4 ml/tube) 10 minutes à 37°C, soit avec 10 µl de DMSO (témoin), soit avec des concentrations variées de composés à tester en solution dans 10 µl de DMSO.

L'incubation est faite en présence de $^{14}C$-Arachidonate (1 µg 0,2 µCi) réparti dans 0,1 ml de tampon II puis on bloque l'incubation au bout de 15 minutes par 50 µl d'acide citrique et 250 µl de NaCl saturé.

Après extraction par l'acétate d'éthyle les métabolites du $^{14}C$-Arachidonate sont séparés par chromatographie sur couche mince et quantifiés à l'aide d'un radio scanner.

Résultats

Le pourcentage d'inhition des voies des prostanoïdes est estimé par mesure de la disparition du thromboxane B2 (TXB2) en fonction de la concentration de chaque composé.

Des résultats correspondant à 50 µM et 100 µM de composés de formule I sont donnés à titre d'exemple dans le tableau II.

**Tableau II**

| Composé n° | Inhibition du LTC4 dans PMNs humains $CI_{50}$(x $10^{-6}$M) | Inhibition du $TxB_2$ dans les plaquettes de lapin (%) 50 µM | 100µM |
|---|---|---|---|
| 1 | 1,2 | 34 | 91 |
| 2 | 0,3 | 100 | 100 |
| 3 | 0,33 | 100 | 100 |
| 4 | 0,25 | 68 | n.d. |
| 5 | 1,6 | 76 | n.d. |
| 6 | 0,2 | 70 | n.d. |
| 7 | n.d. | 36 | 36 |

**2 - Pharmacologie fonctionnelle**

Activité antiinflammatoire mesurée par inhibition de l'afflux protéique dans la chambre antérieure de l'oeil après paracentèse cornéenne

Cette activité est mesurée selon la technique de K. Masuda et coll. (Bibl. Anat. 1977, 6, 99 - 104).

L'effet des diverses concentrations de chaque composé est mesuré sur 6 yeux de lapins Néozélandais albinos.

Les résultats ci-après sont donnés à titre d'exemple.

| Composé | Dose mg/100 ml | Inhibition (%) |
|---|---|---|
| Indométacine | 1 | 80 |
| Composé n° 3 | 0,1 | 20 |
| | 0,5 | 35 |
| | 1 | 50 |

**3 - Toxicité aigüe**

Les rats utilisés sont de souche Wistar d'un poids moyen de 150 g.

La DL50 est déterminée après 14 jours d'observation.

Les résultats ci-après donnent les valeurs de DL50 pour le composé 3 de l'invention accompagnées de l'intervalle de confiance à 5 %.

| | | DL50 (mg/kg) |
|---|---|---|
| Rat mâle | IV | 51 ± 2 |
| | PO | 2g50 ± 90 |
| Rat femelle | IV | 51 ± 2 |
| | PO | 2350 ± 90 |

Les composés de formule I peuvent être utilisés dans le traitement des affections comportant une composante inflammatoires et notamment:

- en ophtalmologie, pour le traitement des inflammations post-traumatiques, des conjonctivites allergiques, des conjonctivites bactériennes, des conjonctivites virales, des blépharites, des uvéites-iridocyclites, des kératites, des kérato-conjonctivites, des rétinites, et des syndrômes de Gougerot-Sjögren,
- en O.R.L., pour le traitement des rhinites allergiques et infectieuses, des rhino-pharyngites des amygdalites, des otites, des stomatites, des gengivites, des alvéolites, des laryngites des epiglotites et des parotidites,
- en dermatologie, dans le traitement du psoriasis, des dermites irritatives et allergiques, des poussées inflammatoires des collagénoses (Lupus, sclérodermies, polyarthrites, dermato-myosis,...), des ulcères et

ulcérations cutanés, de l'acné inflammatoire, des folluculites, des vascularites,

- en pneumologie, dans le traitement des bronchites, des alvéolites, des pleurésies et des pneumopathies,
- en proctologie, dans le traitement des hémorroïdes et des fistules anales,
- en rhumatologie, dans le traitement de l'arthrose, des polyarthrites aiguës ou chroniques, des lombalgies, des sciatiques, des cervicalgies, des tendinites, des inflammations post-traumatiques et des traumatismes musculaires,
- en gynécologie, dans le traitement des bartholinites, des vulvo-vaginites, des prostatites, de l'uréthrite, des cystites, des orchites et des balanites,
- ainsi que dans le traitement des phlébites, des lymphangites et des péricardites.

Les compositions thérapeutiques selon l'invention peuvent être administrées à l'homme ou aux animaux par voie topique orale ou parentérale (y compris par voie intraarticulaire).

Elles peuvent être sous la forme de préparation solides, semi-solides ou liquides. Comme exemples, on peut citer les comprimés, les gélules les suppositoires les solutions ou suspensions injectables, les pommades, les collyres huileux ou aqueux, les collutoires, les solutions nasales et otologiques, ainsi que les formes retard.

Dans ces compositions le principe actif est généralement mélangé avec un ou plusieurs excipients pharmaceutiquement acceptables habituels bien connus de l'homme de l'art.

Les compositions thérapeutiques administrables par voie topique peuvent contenir notamment de 0,1 à 5 % en poids de principe actif.

Les compositions thérapeutiques administrables par voie orale ou parentérale peuvent contenir notamment de 1 à 60 % en poids de principe actif.

La quantité de principe actif administrée dépend évidemment du patient qui est traité, de la voie d'administration et de la sévérité de la maladie. Cependant, pour l'administration par la voie orale ou parentérale on peut administrer environ de 0,25 à 5 mg/kg/jour, soit chez un homme de 70 kg de 17,5 à 350 mg/jour et, de préférence de 25 à 250 mg/jour.

Les composés de formule I et leurs sels d'addition avec des acides phatmaceutiquement acceptables sont également utilisables en raison de leurs propriétés antioxydantes comme conservateurs et antioxydants en alimentation humaine et animale.

On donnera ci-après des exemples de compositions thérapeutiques selon l'invention.

**Solution**

| | |
|---|---|
| Chlorhydrate d'(hydroxy-2 méthyl-4 phényl) amino 2-thiazole | 0,1 % |
| Soluté isotonique | qsp 100 |

**Aérosol topique ORL**

| | |
|---|---|
| Chlorhydrate d'(hydroxy-2 méthyl-4 phényl) amino-2 thiazole | 0,2 % |
| Alcool éthylique à 95° | 2 % |
| Excipient aromatisé | qsp 100 |
| Pressurisation sous pression d'azote | |

**Pommade**

| | |
|---|---|
| Chlorhydrate d'(hydroxy-2 méthyl-4 phényl) amino-2 thiazole | 5 % |
| Huile de vaseline | 20 % |
| Vaseline | 75 % |

**Comprimé**

| | |
|---|---|
| Chlorhydrate d'(hydroxy-2 méthyl-4 phényl) amino-2 thiazole | 0,100 g |
| Amidon de Maïs | 0,075 g |
| Stéarate de Magnésium | 0,020 g |
| Acide silicique colloïdal, | 0,005 g |

**Gélule**

| | |
|---|---|
| Chlorhydrate d'(hydroxy-2 méthyl-4 phényl) amino-2 thiazole | 0,100 g |
| Stéarate de Magnésium | 0,020 g |

**Suppositoire**

| | |
|---|---|
| Chlorhydrate d'(hydroxy-2 méthyl-4 phényl) amino-2 thiazole | 0,200 g |
| Glycérides semi-synthétiques qsp. environ | 1,700 g |

**Revendications** pour les Etats Contractants: BE, CH, LI, DE, FR, GB, IT, LU, NL, SE

1. Composés de formule:

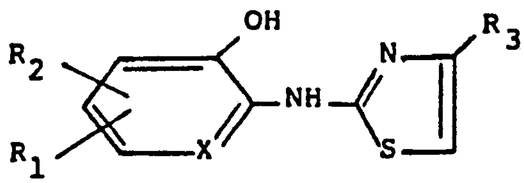

(I)

dans laquelle:

X représente un groupe C-H ou un atome d'azote,

$R_1$ et $R_2$ représentent indépendamment l'un de l'autre un atome d'hydrogène, un atome d'halogène, un radical alkyle en $C_1$-$C_{12}$, un radical alkoxy en $C_1$-$C_6$, un radical alkylthio en $C_1$-$C_6$ un radical alkenyloxy en $C_2$-$C_6$, un radical cycloalkyle en $C_5$-$C_7$, un radical trifluorométhyle, un groupe nitro, un groupe amino, un groupe (alkyl en $C_1$-$C_6$) amino ou un groupe di(alkyl en $C_1$-$C_6$) amino,

$R_3$ représente un atome d'hydrogène, un groupe $-CH_2OH$ ou un groupe $-COOR_4$, $R_4$ étant un atome d'hydrogène ou un groupe alkyle en $C_1$-$C_6$,

ainsi que leurs sels pharmaceutiquement acceptables.

2. Composés selon la revendication 1, caractérisé en ce que $R_1$ est un atome d'hydrogène, un atome d'halogène en position 5, un groupe alkyle en $C_1$-$C_4$ en position 4 ou 5 et $R_2$ est un atome d'hydrogène, ou bien dans lesquels $R_1$ et $R_2$ sont des groupes alkyle en $C_1$-$C_4$ en positions 3 et 5.

3. Composés selon la revendication 2, caractérisé en ce que X est CH, $R_1$ est un radical 4-méthyle et $R_2$ est un atome d'hydrogène.

4. Composés selon la revendication 3 qui sont l'(hydroxy-2 méthyl-4 phényl) amino-2-thiazole et ses sels pharmaceutiquement acceptables.

5. Procédé de préparation d'un composé selon la revendication 1, caractérisé en ce que l'on fait réagir une thiourée N-substituée de formule:

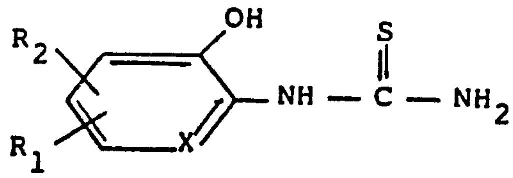

(II)

dans laquelle $R_1$, $R_2$ et X ont la signification donnée ci-dessus avec du dichloro-1,2 éthoxy-2 éthane du chloroacétaldéhyde, du chloroacétaldéhyde diéthylacétal de l'acétate de dichloro-1,2 éthyle ou un bromopyruvate d'alkyle en $C_1$-$C_6$ et pour obtenir un composé de formule I dans laquelle $R_3$ est un groupe -COOH on effectue une saponification d'un ester correspondant et pour obtenir un composé de formule I dans laquelle $R_3$ est un groupe $-CH_2OH$ on effectue une réduction de cet ester.

6. Composition thérapeutique, caractérisé en ce qu'elle contient à titre de principe actif un composé selon l'une quelconque des revendications 1 à 4.

7. Composition thérapeutique selon la revendication 6, caractérisée en ce qu'elle est sous une forme administrable par voie topique.

8. Composition thérapeutique selon la revendication 6, caractérisée en ce qu'elle est sous une forme administrable par voie orale.

9. Composition thérapeutique selon la revendication 6, caractérisée en ce qu'elle est sous une forme administrable par voie parentérale.

**Revendications** pour l'Etat Contractant: AT

1. Procédé de préparation de composés de formule:

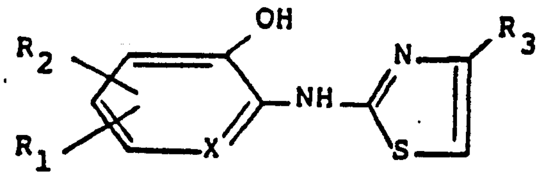

(I)

dans laquelle:

X représente un groupe C-H ou un atome d'azote,

$R_1$ et $R_2$ représentent indépendamment l'un de l'autre un atome d'hydrogène un atome d'halogène, un radical alkyle en $C_1$-$C_{12}$, un radical alkoxy en $C_1$-$C_6$, un radical alkylthio en $C_1$-$C_6$, un radical alkenyloxy en $C_2$-$C_6$, un radical cycloalkyle en $C_5$-$C_7$, un radical trifluorométhyle, un groupe nitro, un groupe amino, un groupe (alkyl en $C_1$-$C_6$) amino ou un groupe di(alkyl en $C_1$-$C_6$) amino,

$R_3$ représente un atome d'hydrogène, un groupe $-CH_2OH$ ou un groupe $-COOR_4$, $R_4$ étant un atome d'hydrogène ou un groupe alkyle en $C_1$-$C_6$,

ainsi que de leurs sels pharmaceutiquement acceptables, caractérisé en ce que l'on fait réagir une thiourée N-substituée de formule:

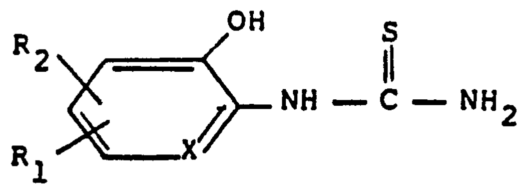

(II)

dans laquelle $R_1$, $R_2$ et X ont la signification donnée ci-dessus avec du dichloro-1,2 éthoxy-2 éthane, du chloroacétaldéhyde, du chloroacétaldéhyde diéthylacétal, de l'acétate de dichloro-1,2 éthyle ou un bromopyruvate d'alkyle en $C_1$-$C_6$ et pour obtenir un composé de formule I dans laquelle $R_3$ est un groupe -COOH on effectue une saponification d'un ester correspondant et pour obtenir un composé de formule I dans laquelle $R_3$ est un groupe $-CH_2OH$ on effectue une réduction de cet ester.

2. Procédé selon la revendication 1 caractérisé en ce que l'on prépare un composé de formule I dans laquelle $R_1$ est un atome d'hydrogène, un atome d'halogène en position 5, un groupe alkyle en $C_1$-$C_4$ en position 4 ou 5 et $R_2$ est un atome d'hydrogène, ou bien dans lesquels $R_1$ et $R_2$ sont des groupes alkyle en $C_1$-$C_4$ en position 3 et 5.

3. Procédé selon la revendication 2, caractérisé en ce que l'on prépare un composé de formule I dans laquelle X est CH, $R_1$ ets un radical 4-méthyle et $R_2$ est un atome d'hydrogène.

4. Procédé selon la revendication 3, dans lequel on prépare l'(hydroxy-2 méthyl-4 phényl) amino-2-thiazole ou ses sels pharmaceutiquement acceptables.

5. Procédé de préparation d'une composition pharmaceutique, comprenant le mélange d'une quantité efficace d'un composé tel que défini dans la revendication 1 avec au moins un véhicule pharmaceutiquement acceptable.

**Patentansprüche** für die Vertragsstaaten: BE, CH, LI, DE, FR, GB, IT, LU, NL, SE

1. Verbindungen der Formel (I):

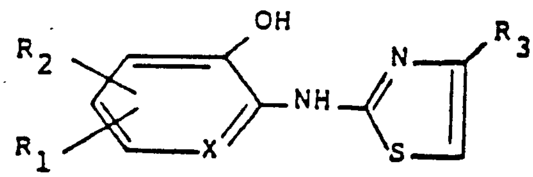

(I)

in der bedeuten:

X eine CH-Gruppe oder ein Stickstoffatom,

$R_1$ und $R_2$, unabhängig voneinander, Wasserstoff oder Halogen, $C_{1-12}$-Alkyl, $C_{1-6}$-Alkoxy, $C_{1-6}$-Alkylthio, $C_{2-6}$ Alkenyloxy $C_{5-7}$-Cycloalkyl Trifluormethyl Nitro, Amino, $C_{1-6}$-Alkylamino oder Di-$C_{1-6}$-alkylamino,

$R_3$ Wasserstoff, -$CH_2OH$ oder -$COOR_4$ mit $R_4$=Wasserstoff oder $C_{1-6}$-Alkyl,

sowie ihre pharmazeutisch verträglichen Salze.

2. Verbindungen nach Anspruch 1, dadurch gekennzeichnet, daß in Formel (I), $R_1$ Wasserstoff oder Halogen in Stellung 5, $C_{1-4}$-Alkyl in Stellung 4 oder 5 und $R_2$ Wasserstoff ist, oder in der $R_1$ und $R_2$ $C_{1-4}$-Alkyl in Stellung 3 und 5 sind.

3. Verbindungen nach Anspruch 2, dadurch gekennzeichnet, daß in Formel (I) X CH, $R_1$ 4-Methyl und $R_2$ Wasserstoff ist.

4. Verbindungen nach Anspruch 3, gekennzeichnet durch 2-(2-Hydroxy-4-methylphenyl)-aminothiazol und seine pharmazeutisch verträglichen Salze.

5. Verfahren zur Herstellung einer Verbindung nach Anspruch 1, gekennzeichnet durch Umsetzung eines N-substituierten Thioharnstoffs der Formel (II):

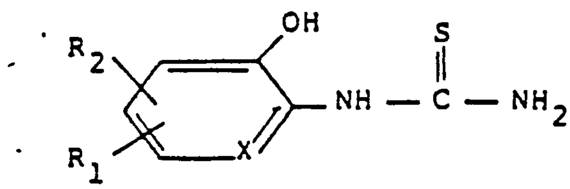

(II)

in der $R_1$, $R_2$ und X die oben angegebene Bedeutung haben,

mit 1,2-Dichlor-2-ethoxyethan, Chloracetaldehyd, Chloracetaldehyd-diethylacetal, 1,2-Dichlorethylacetat oder $C_{1-6}$-Alkylbrompyruvat,

zur Herstellung einer Verbindung der Formel (I), in der $R_3$ -COOH ist, Verseifung eines entsprechenden Esters

und

zur Herstellung einer Verbindung der Formel (I), in der $R_3$ -$CH_2OH$ ist, Reduktion dieses Esters.

6. Therapeutische Zusammensetzung, gekennzeichnet durch eine Verbindung nach einem der Ansprüche 1 bis 4 als Wirkstoff.

7. Therapeutische Zusammensetzung nach Anspruch 6 in einer äußerlich verabreichbaren Form.

8. Therapeutische Zusammensetzung nach Anspruch 6 in oral verabreichbarer Form.

9. Therapeutische Zusammensetzung nach Anspruch 6 in 10 parenteral verabreichbarer Form.

**Patentansprüche** für den Vertragsstaat: AT

1. Verfahren zur Herstellung von Verbindungen der Formel (I):

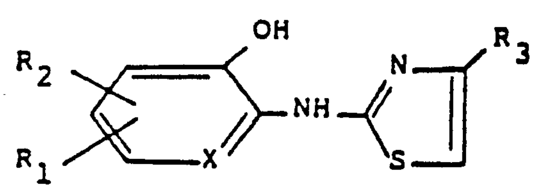

(I)

in der bedeuten:

X eine CH-Gruppe oder ein Stickstoffatom,

$R_1$ und $R_2$, unabhängig voneinander, Wasserstoff, Halogen, $C_{1-12}$-Alkyl, $C_{1-6}$-Alkoxy, $C_{1-6}$-Alkylthio, $C_{2-6}$-Alkenyloxy, $C_{5-7}$-Cycloalkyl, Trifluormethyl, Nitro, Amino, $C_{1-6}$-Alkylamino oder Di-$C_{1-6}$-alkylamino,

$R_3$ Wasserstoff, $-CH_2OH$ oder $-COOR_4$ mit $R_4$ = Wasserstoff oder $C_{1-6}$-Alkyl,

sowie ihrer pharmazeutisch verträglichen Salze,

gekennzeichnet durch

Umsetzung eines N-substituierten Thioharnstoffs der Formel (II):

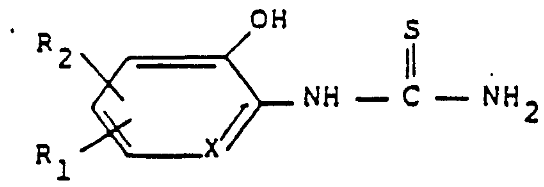
(II)

in der $R_1$, $R_2$ und X die oben angegebene Bedeutung haben, mit 1,2-Dichlor-2-ethoxyethan, Chloracetaldehyd, Chloracetaldehyd-diethylacetal, 1,2-Dichlorethylacetat oder $C_{1-6}$-Alkylbrompyruvat,

zur Herstellung einer Verbindung der Formel (I), in der $R_3$ -COOH ist, Verseifung eines entsprechenden Esters

und

zur Herstellung einer Verbindung der Formel (I), in der $R_3$ $-CH_2OH$ ist, Reduktion dieses Esters.

2. Verfahren nach Anspruch 1, gekennzeichnet durch die Herstellung einer Verbindung der Formel (I), in der $R_1$ Wasserstoff oder Halogen in Stellung 5, $C_{1-4}$-Alkyl in Stellung 4 oder 5 und $R_2$ Wasserstoff ist, oder in der $R_1$ und $R_2$ $C_{1-4}$-Alkyl in Stellung 3 und 5 sind.

3. Verfahren nach Anspruch 2, gekennzeichnet durch Herstellung einer Verbindung der Formel (I), in der X CH, $R_1$ 4-Methyl und $R_2$ Wasserstoff ist.

4. Verfahren nach Anspruch 3, gekennzeichnet durch Herstellung von 2-(2-Hydroxy-4-methylphenyl)-aminothiazol oder seiner pharmazeutisch verträglichen Salze.

5. Verfahren zur Herstellung einer pharmazeutischen Zusammensetzung gekennzeichnet durch Vermischen einer wirkungsvollen Menge einer Verbindung gemäß Anspruch 1 mit mindestens einem pharmazeutisch verträglichen Träger.

**Claims** for the Contracting States: BE, CH, LI, DE, FR, GB, IT, LU, NL, SE

1. Compounds of formula:

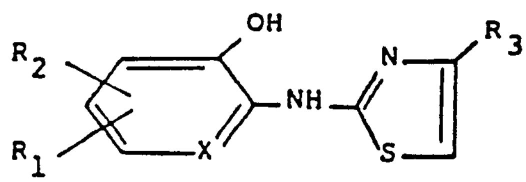
(I)

wherein:

X represents a C-H group or a nitrogen atom,

$R_1$ and $R_2$ represent, independently of each other, a hydrogen atom, a halogen atom, a $C_1$-$C_{12}$ alkyl radical, a $C_1$-$C_6$ alkoxy radical, a $C_1$-$C_6$ alkylthio radical, a $C_2$-$C_6$ alkenyloxy radical, a $C_5$-$C_7$ cycloalkyl radical, a trifluoromethyl radical, a nitro group, an amino group, a ($C_1$-$C_6$ alkyl)amino group or a di($C_1$-$C_6$ alkyl)amino group,

$R_3$ represents a hydrogen atom, a $-CH_2OH$ group or a $-COOR_4$ group, $R_4$ being a hydrogen atom or a $C_1$-$C_6$ alkyl group,

and also the pharmaceutically acceptable salts thereof.

2. Compounds as claimed in claim 1, characterized in that $R_1$ is a hydrogen atom, a halogen atom in the 5-position or a $C_1$-$C_4$ alkyl group in the 4- or 5-position and $R_2$ is a hydrogen atom, or alternatively in which $R_1$ and $R_2$ are $C_1$-$C_4$ alkyl groups in the 3- and 5- positions.

3. Compounds as claimed in claim 2, characterized in that X is CH, $R_1$ is a 4-methyl radical and $R_2$ is a hydrogen atom.

4. Compounds as claimed in claim 3 being 2-[(2-hydroxy-4-methyl-phenyl)-amino]thiazole and the pharmaceutically acceptable salts thereof.

5. A process for preparing a compound as claimed in claim 1, characterized in that a N-substituted thiourea of formula:

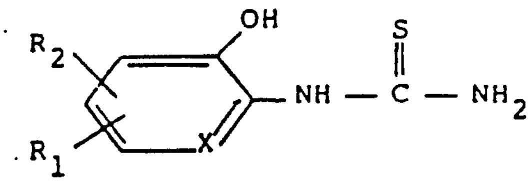

(II)

wherein $R_1$, $R_2$ and X have the meaning given above, is reacted with 1,2-dichloro-2-ethoxyethane, chloroacetaldehyde, chloroacetaldehyde diethyl acetal, 1,2-dichloroethyl acetate or a $C_1$-$C_6$ alkyl bromopyruvate and, to obtain a compound of formula I wherein $R_3$ is a -COOH group, saponification of a corresponding ester is effected and, to obtain a compound of formula I wherein $R_3$ is a $-CH_2OH$ group, this ester is reduced.

6. A therapeutic composition, characterized in that it comprises as active ingredient a compound as claimed in any one of claims 1 to 4.

7. A therapeutic composition as claimed in claim 6, characterized in that it is in a form which can be administered topically.

8. A therapeutic composition as claimed in claim 6, characterized in that it is in a form which can be administered orally.

9. A therapeutic composition as claimed in claim 6 characterized in that it is in a form which can be administered parenterally.

**Claims** for the Contracting State: AT

1. A process for preparing compounds of formula:

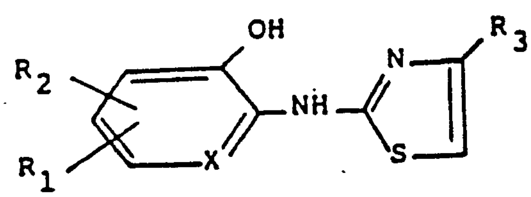

(I)

wherein:

X represents a C-H group or a nitrogen atom,

$R_1$ and $R_2$ represent, independently of each other, a hydrogen atom, a halogen atom, a $C_1$-$C_{12}$ alkyl radical, a $C_1$-$C_6$ alkoxy radical, a $C_1$-$C_6$ alkylthio radical, a $C_2$-$C_6$ alkenyloxy radical, a $C_5$-$C_7$ cycloalkyl radical, a trifluoromethyl radical, a nitro group, an amino group, a ($C_1$-$C_6$ alkyl)amino group or a di($C_1$-$C_6$ alkyl)amino group,

$R_3$ represents a hydrogen atom, a $-CH_2OH$ group or a $-COOR_4$ group, $R_4$ being a hydrogen atom or a $C_1$-$C_6$ alkyl group,

and also the pharmaceutically acceptable salts thereof, characterized in that a N-substituted thiourea of formula:

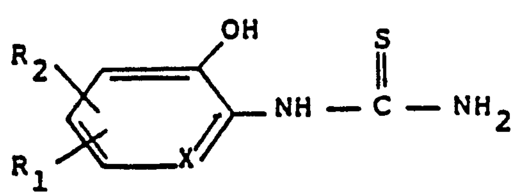

(II)

wherein $R_1$, $R_2$ and X have the meaning given above is reacted with 1,2-dichloro-2-ethoxyethane, chloroacetaldehyde, chloroacetaldehyde diethyl acetal, 1,2-dichloroethyl acetate or a $C_1$-$C_6$ alkyl bromopyruvate and, to obtain a compound of formula I wherein $R_3$ is a -COOH group, saponification of a corresponding ester is effected and, to obtain a compound of formula I wherein $R_3$ is a $-CH_2OH$ group, this ester is reduced.

2. A process as claimed in claim 1, characterized in that a compound of formula I is prepared wherein $R_1$ is a hydrogen atom, a halogen atom in the 5-position, or a $C_1$-$C_4$ alkyl group in the 4- or 5-position and $R_2$ is a hydrogen atom, or alternatively in which $R_1$ and $R_2$ are $C_1$-$C_4$ alkyl groups in the 3- and 5-positions.

3. A process as claimed in claim 2, characterized in that a compound of formula I is prepared wherein X is CH, $R_1$ is a 4-methyl radical and $R_2$ is a hydrogen atom.

4. A process as claimed in claim 3, wherein the compound prepared is 2-[(2-hydroxy-4-methyl-phenyl)-amino]thiazole or any pharmaceutically acceptable salt thereof.

5. A process for preparing a pharmaceutical composition, comprising admixture of an effective quantity of a compound as defined in claim 1 with one or more pharmaceutically acceptable carrier.